Europäisches Patentamt

European Patent Office

Office européen des brevets

(11) Veröffentlichungsnummer: **0 417 531 A1**

# (12) EUROPÄISCHE PATENTANMELDUNG

(21) Anmeldenummer: 90116305.5

(22) Anmeldetag: 25.08.90

(51) Int. Cl.5: **C12N 5/00**, C12N 5/02,
//C12M3/00

(30) Priorität: 09.09.89 DE 3930140

(43) Veröffentlichungstag der Anmeldung:
**20.03.91 Patentblatt 91/12**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Steiner, Ulrich, Dr.**
**Krutscheider Weg 55**
**W-5600 Wuppertal 1(DE)**
Erfinder: **Seifert, Horst, Prof. Dr.**
**Heinrich-Deppe-Ring 28**
**W-3406 Bovenden 1(DE)**
Erfinder: **Böhnel, Helge, Dr.**
**Birkenweg 2a**
**W-3406 Bovenden 1(DE)**
Erfinder: **Roth, Frauke, Dr.**
**Kookweg 5**
**W-3403 Friedland(DE)**

(54) Verfahren zur Herstellung von biologischen Materialien in Zellkulturen und Vorrichtungen.

(57) Die Erfindung betrifft ein Verfahren zur Herstellung von biologischen Materialien in Zellkulturen in einer kontinuierlichen Fermentationsweise und einer Vorrichtung zur Durchführung dieses Verfahrens. Das Verfahren erlaubt es, eine kontinuierliche Fermentation auch dann durchzuführen, wenn der herzustellende Wirkstoff erst nach einer geeigneten Induktion der Zellkultur freigesetzt wird, und auch wenn dies zu einer Zerstörung der induzierten Zellen führte. Unter Induktion wird verstanden die Behandlung mit einem chemischen Agenz oder mit physikalischen Einflußgrößen oder die Infizierung der Zellen z.B. mit einem Virus, oder Kombinationen davon.

EP 0 417 531 A1

# VERFAHREN ZUR HERSTELLUNG VON BIOLOGISCHEN MATERIALIEN IN ZELLKULTUREN UND VORRICHTUNGEN

Bisher konnten biologische Materialien, wie Wirkstoff, Protein, Enzyme etc., die durch Induktion von Kulturen herzustellen sind, nur mit absatzweisen Produktionsverfahren hergestellt werden. Dies bedeutet, daß die Zellen in einem Kulturgefäß wie z.B. einem Fermenter angezogen werden und nach Erreichen eines bestimmten Zustandes die Induktion gesetzt wird. Nach einer vom Wirkstoff abhängigen Zeitspanne - in der Regel einige Tage - kann die Ernte erfolgen. Danach ist der Fermenter abzubrechen, zu reinigen und neu vorzubereiten für die nächste Fermentation. Bevor die nächste Induktion stattfinden kann, muß in einer Wachstumsphase erst wieder die Zellmasse aufgebaut werden. Der Nachteil dieses Verfahrens ist eine geringe Raum-Zeit-Ausbeute bedingt dadurch, daß der gesamte Zyklus Fermentervorbereitung, Wachstumsphase, Wirkphase der Induktion und Fermenterreinigung für jede einzelne Ernte erforderlich ist und daß nur geringe Zellkonzentrationen erreicht werden. Hinzu kommt, daß für große Produktmengen großvolumige Fermenter einzusetzen sind, so daß eine Kontamination den Verlust von großen Mengen an Nährlösung bewirkt.

Diese Nachteile bestehen nicht bei einem kontinuierlichen Produktionsverfahren. Nach der einmaligen Fermentervorbereitung und Wachstumsphase kann der Fermentationsprozeß langfristig ohne Unterbrechung laufen und es wird eine wesentlich höhere Zellkonzentration erreicht. Dadurch steigt die Raum-Zeit-Ausbeute was die Reduktion der Volumen der Fermentergefäße erlaubt. Entsprechend sinken die Materialverluste im Falle einer Kontamination. Solche Verfahren wurden bisher erfolgreich eingesetzt für die Herstellung von Wirkstoffen aus Zellkulturen, wobei die Zellen den Wirkstoff oder Vorstufen davon synthetisieren und in die Nährlösung abgeben, ohne daß eine Induktion erforderlich ist. Beispiele sind Zellkulturen von Bowes Melanoma Zellen (Produkt: Tissue Plasminogen Activator), Hybridoma Zellen (Produkt: Monoclonale Antikörper) oder Zellen mit neukombinierter Nukleinsäure (Produkt: z.B. Faktor VIII). Ein weiterer Vorteil der kontinuierlichen Kultur ist die bessere Qualität des Produktes, da sich hier im Gegensatz zur absatzweisen Produktion konstante Kulturbedingungen einstellen lassen z.B. bezüglich Zelldichte, Konzentration der Nährstoffe und Konzentration von Abbauprodukten und unerwünschten Beiprodukten wie Proteasen. Wegen der geringeren Verweilzeit des Produktes in der Nährlösung iat zudem auch der Abbau des Produktes durch z.B. Proteasen oder anderen chemischen Wechselwirkungen in der Nährlösung reduziert.

Mit dem Verfahren der Erfindung wird eine kontinuierliohe Produktion nun auch bei Produktionsverfahren möglich, die eine Induktion der Kultur erfordern.

Unterschiedliche Arten der Induktion lassen sich mit der Erfindung realisieren:

## 1. Induktion durch Infektion mit einem anderen Organismus

Beispiel für einen anderen Organismus sind Viren. Zur Herstellung von Virus und viralem Antigen wird die Induktion der Kultur durchgeführt durch Infektion mit dem Virus. Statt eines natürlichen Virus kann auch ein Virus verwendet werden, der mit Hilfe von gentechnischen Methoden so verändert wurde, daß die infizierten Zellen ein rekombinantes Protein exprimieren. Von besonderem Interesse sind hier Kulturen von Insektenzellen und ein Virusexpressionssystem wie z.B. das Baculovirus-Expressionssystem. Diese Expressionssyteme zeichnen sich dadurch aus, daß die Viren nicht infektiös sind für Menschen und somit eine große Sicherheit bei der Anwendung von in diesen Systemen exprimierten rekombinanten Proteinen am Menschen besteht. Außerdem ist gezeigt worden, daß mit diesen Systemen hohe Produktkonzentrationen in der Nährlösung erreicht werden können, was sich sowohl auf die Ökonomie wie auch - wegen der einfacheren Reinigung bei höheren Konzentrationen - auf die Produktqualität positiv auswirkt. Bisher sind jedoch nur absatzweise Produktionsverfahren eingesetzt worden, die die oben beschriebenen Nachteile haben.

## 2. Induktion durch vorübergehende oder permanente Veränderung der chemischen Bedingungen

Hier wird die Induktion erzielt, indem das chemische Milieu, in dem sich die Zellen befinden, geändert wird. In der Regel wird diese Veränderung durch Zugabe von Substanzen erreicht. Beispiel für einen solchen Prozeß ist die Produktion von Lymphokinen wie IL-2 mit tierischen Zellkulturen. Hier wird die Induktion erreicht, indem Phorbolester der Nährlösung zugegeben wird.

Eine vorübergehende Änderung läßt sich erzielen, wenn die Milieuänderung rückgängig gemacht werden kann, z.B. in dem die zugegebenen Substanzen durch Fällung oder Inaktivierung oder Abzug z.B. über Membransysteme entfernt werden.

3. Induktion durch vorübergehende oder permanente Veränderung der physikalischen Bedingungen

Verändert werden können die physikochemischen Bedingungen der Nährlösung wie z.B. pH, $pO_2$ Redoxpotential, Viskosität als auch physikalische Bedingungen wie Temperatur, Einwirkung von Licht und anderer Strahlung, Einwirkung von elektrischen Strömen und Magnetfeldern, und anderes mehr. Als Beispiel sei die Herstellung von Heat-Shock-Proteinen genannt, wofür die Zellen vorübergehend einer höheren Temperatur ausgesetzt werden.

Beschreibung des Verfahrens

Die Aufgabe der Erfindung, eine kontinuierliche Kultur in Prozessen, die eine Induktion erfordern, zu ermöglichen, wurde wie folgt gelöst:
A) Wenn die Induktion dauerhaft bestehen darf oder muß:
In einem Bioreaktor A werden Zellen in Suspension in einem Nährmedium kultiviert. Nach Erreichen einer bestimmten Zelldichte in Bioreaktor A wird begonnen, die Suspension in einen zweiten Bioreaktor B kontinuierlich überzuleiten und gleichzeitig frisches Nährmedium in den Bioreaktor A so einzuleiten, daß dessen Füllvolumen im zeitlichen Mittel konstant bleibt. Die Zelldichte und Wachstumsrate der Zellen im Bioreaktor A kann eingestellt werden durch den Volumenstrom, mit dem Suspension von Bioreaktor A nach Bioreaktor B überführt wird. Die Zellen werden in dem Bioreaktor B induziert. Zur Optimierung der Wachstums- und Produktbildungsbedingungen kann ein Erhaltungsmedium zusätzlich in den Bioreaktor B kontinuierlich zugegeben werden. Der Inhalt des Bioreaktors B, der aus der verbrauchten Nährlösung, den restlichen Zellen, den Zellfragmenten, dem Induktor und weiteren Stoffen besteht, wird kontinuierlich geerntet. Die mittlere Verweilzeit in dem Bioreaktor B muß im Hinblick auf optimale Produktqualität und Produktausbeute experimentell bestimmt werden. Für Induktoren, die zu einer Lyse der Zellen führen, mag dies z.B. der Zustand sein, bei dem 80 % der Zellen lysiert sind. Die Verweilzeit kann durch Wahl des Volumens des Bioreaktors B eingestellt werden.
B) Wenn die Induktion nur vorübergehend sein soll:
Die Induktion sollte dann nicht im Bioreaktor B stattfinden, sondern in der Transferleitung, die von Bioreaktor A nach Bioreaktor B führt, vorgenommen werden und bei Eintritt der Zellen in den Bioreaktor B abgeschlossen sein. Die Dauer der vorübergehenden Induktion kann gesteuert werden durch die Verweilzeit in der Transferleitung, die durch geeignete Versuche ermittelt werden kann und die durch Länge und Form der Leitung sowie durch den Volumenstrom eingestellt werden kann. Ansonsten entspricht das Verfahren dem wie für eine dauerhafte Induktion beschriebenen.
Die Aufreinigung der aus dem Bioreaktor B kontinuierlich entnommenen Ernten erfolgt entweder kontinuierlich oder - nach Sammlung in einem Sammelgefäß - absatzweise. Es werden bekannte Trennverfahren eingesetzt, wie Trennung über Membranen verschiedener Porengröße, Zentrifugen, Säulentrennverfahren u.a. Die Reinigungsprozedur hängt ab von den eingesetzten Zellkulturen und Nährlösungen, den Induktionen, dem Produkt und den Anforderungen an die Reinheit des Produktes. Die Renigungsprozedur ist unter Umständen für jedes Produkt neu zu bestimmen.
Die Meß- und Regeltechnik für die Bioreaktoren und Pumpen wird nach dem Stand der Technik ausgelegt. Vorzugsweise wird die Steuerung der Gesamtanlage durch einen übergeordneten Leitrechner vorgenommen jedoch können auch individuelle Regler eingesetzt werden.
Die Steuerung der Pumpraten für den Transfer von Nährlösung und Zellsuspension zwischen den Vorrats-, Fermenter- und Erntegefäßen erfolgt vorzugsweise durch photometrische Bestimmung der Zelldichte bzw. des Ausmaßes der Lyse der Zellkultur.
Die Begasung der Bioreaktoren kann erfolgen durch direktes Einblasen z.B. von Luft, $O_2$, $N_2$ oder $CO_2$ in die Flüssigkeit oder indirekt durch Ausnutzung der Diffusion über die Oberfläche der Flüssigkeit oder durch Membranen (Hohlfasermembranen oder Silikonschlauchmembranen), die in die Flüssigkeit der Bioreaktoren eintauchen oder in einer externen Schleife eingebaut sind.
Zur Vermeidung von Kontaminationen vorgeschalteter Systeme durch Inhaltsstoffe der Folgesysteme werden vorzugsweise alle Verbindungen zwischen den Systemen mit Rückwuchsfallen ausgestattet.

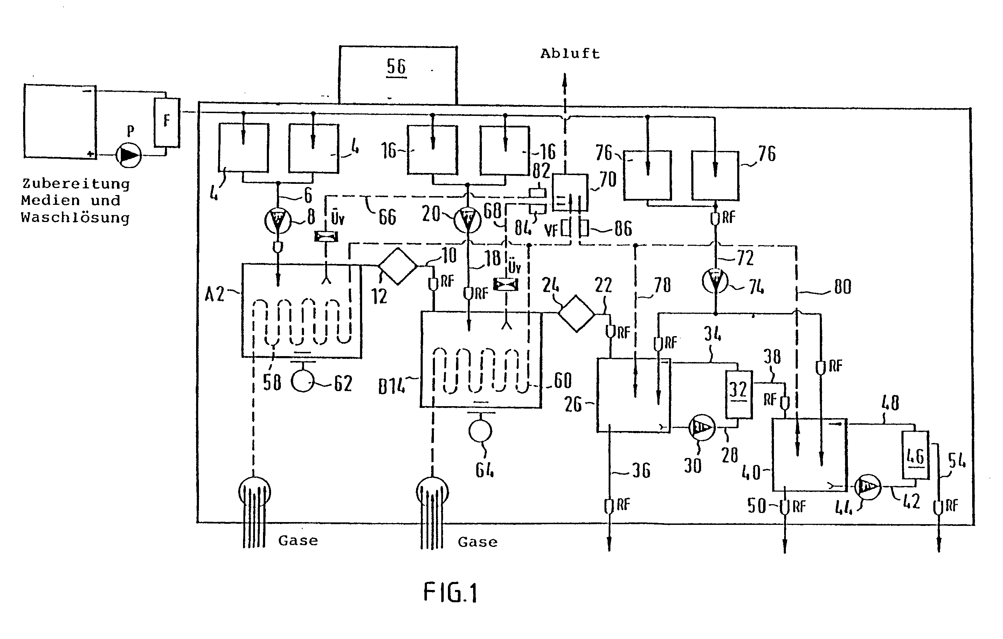

Die Figur 1) zeigt eine erfindungsgemäß bevorzugte Vorrichtung mit einem Biorektor A 2, in den gesteuert aus Nährmediumvorratsbehältern 4 über eine Leitung 6, die mit einer Pumpe 8 ausgerüstet ist, Nährmedium in den Bioreaktor A 2 eingeführt wird.

Der Bioreaktor A 2 ist hier weiter mit Hohlfasermembranen 58 ausgerüstet, über die Gase zur Regulierung des pH oder $pO_2$ eingeleitet werden können. Zusätzlich ist der Bioreaktor A 2 mit einem Magnetrührer 62 und über eine Leitung 66, die ein Überdruckventil aufweist, mit dem Abluftsicherheitsgefäß 70 verbunden, wobei die Leitung einen Hohlfaserfilter 82 zum Zurückhalten eventuell vorhandener Viren aufweist. Der Bioreaktor A 2 ist über eine Leitung 10, die mit einem Photometer 12 und einer Rückwuchsfalle ausgerüstet ist, mit dem Bioreaktor B 14 verbunden, in den über eine Leitung 18, die mit einer Pumpe 20 und einer Ruckwuchsfalle ausgerüstet ist, aus den Behältern für Erhaltungsmedium 16 Medium zum Bioreaktor B 14 zugeführt wird. Weiter ist der Bioreaktor B 14 mit einer Hohlfasermembran 60 ausgerüstet, die zur Begasung und mit zur Regulierung des pH und $pO_2$ dient. Zusätzlich weist der Bioreaktor B 14 einen Magnetrührer 64 zum Vermischen der Inhaltsstoffe auf. Eine Leitung 68 mit einem Überdruckventil dient zum Druckausgleich und führt in das Abluftsicherheitsgefäß 70 über einen Hohlfaserfilter 84, in dem eventuell vorhandene Viren zurückgehalten werden. Der Bioreaktor B 14 ist mit dem ersten Retentatbehälter 26 der ersten Filtrationsanordnung über eine Leitung 22, die mit einem Photometer 24 und einer Rückwuchsfalle ausgerüstet ist, verbunden, der wiederum über eine Leitung 78 mit einem daran vorhandenen Hohlfaserfilter 86 mit dem Abluftsicherheitsgefäß 70 zum Druckausgleich verbunden ist. Retentat aus dem ersten Retentatbehälter 26 wird über die Leitung 36, die mit einer Rückwuchsfalle ausgerüstet ist, abgezogen. hus dem ersten Retentatbehälter 26 wird über eine Leitung 28, die mit einem Photometer 30 ausgerüstet ist, Suspension in den ersten Filter 32 geleitet, und daraus das Retentat über die Leitung 34 in den ersten Retentatbehälter 26 zurückgeführt und das Filtrat über die Leitung 38, die mit einer Rückwuchsfalle ausgerüstet ist, in den zweiten Retentatbehälter 40 eingeleitet, der wiederum mit einer Druckausgleichsleitung 80 versehen ist, die über einen Hohlfaserfilter 86 in das Abluftsicherheitsgefäßt 70 mündet. Weiter ist der zweite Retentatbehälter 40 mit einer Leitung 50 versehen, die eine Rückwuchsfalle enthält und über die Viruskonzentrat abgezogen wird. Aus dem zweiten Retentatbehälter 40 wird über eine Leitung 42, die mit einer Pumpe 44 versehen ist, Suspension in die Filtrationsvorrichtung 46 geführt, deren Retentat über die Leitung 48 in den zweiten Retentatbehälter 40 zurückgeführt wird, und deren Filtrat über die Leitung 54, die mit einer Rückflußfalle ausgestattet ist, abgezogen wird. Aus den Waschflüssigkeitsbehältern 76 kann ebenfalls rechnergesteuert über Leitungen 72, die mit Rückflußfallen ausgestattet sind und mit einer Pumpe 74 versehen sind, Waschflüssigkeit in die Retentatbehälter 26, 40 zugeleitet werden.

Die Steuerung der Zu- und Ableitungen zu und aus den verschiedenen Teilen der Vorrichtung wird mit Hilfe eines Prozeßrechners 56 durchgeführt.

Das folgende Beispiel soll die Erfindung weiter erläutern.

Beispiel 1

Herstellung von BHK 21 Klon 13 Zellen in kontinuierlicher Suspensionskultur.

Die Anzucht der Zellen BHK 21 Klon 13 (ECACC Nr. 84100501 (zu beziehen durch Flow Laboratories Meckenheim)) kann sowohl in Monolayer- als auch in Rollerflaschen erfolgen. Zur Inokulation müssen im Fermenter mindestens $8 \times 10^4$ Zellen pro ml vorhanden sein, um ein schnelles Anwachsen der Kultur zu gewährleisten. Die Verdopplungszeit der BHK 21 Klon 13 Zellen betrug 24 Stunden. Mit der kontinuierlichen Zellgewinnung konnte nach 3 Tagen begonnen werden, sobald die Zellzahl mindestens $4 \times 10^5$ pro ml betrug. Die Mediumzugabe (9,538 g/l MEM (minimal ess. media) mit Earls Spinnersalzen, 2,2 g/l $NaHCO_3$, 10,0 ml/l MEM-Vitaminlösung, 10,0 ml/l nicht essentielle Aminosäuren, 10,0 %/l foetales Kälberserum, über Hohlfaserbündel, die direkt an den Bioreaktor angeschlossen sind, steril filtriert) betrug zum Fermentationsbeginn 240 ml pro Tag, das entspricht einer Verdünnung von 1:12,5 und konnte proportional zum Wachstum bis 1500 ml pro Tag gesteigert werden (1:2). Die Regulierung erfolgte über ein eingebautes Photometer. Die Zellen wuchsen ohne Mikrocarrier Das Reaktionsvolumen des Bioreaktors A betrug 4 l, das Flüssigkeitsvolumen 3 l. Die Reaktionstemperatur war 37°C, die Rührgeschwindigkeit 150 U/min, der pH-Wert wurde auf 7,0 und der $pO_2$ auf 45 % $O_2$ einer luftkalibrierten Lösung eingestellt. Der Gasgrundstrom betrug hierbei 4,5 l/min. Als Vordruck wurden 40 mbar $N_2$, 20 mbar $O_2$ und 30 mbar $CO_2$ sowie Luft ohne Regelung eingesetzt. Als Geräte wurden im Beispiel die Folgenden eingesetzt:

Hersteller:

|  |  |  |
|---|---|---|
| Mediumzugabe: | Pumpe IV | BCC |
|  |  | R. Wissel Str. 11 |
|  |  | D-3400 Göttingen |
|  | Pumpe III | BCC |
|  |  |  |
| Temperaturregelung: | Modul TEM | BCC |
| Temperaturfühler: | PT 100 | Schraer |
|  |  | Postfach 747 |
|  |  | D-4920 Lemgo 1 |
| Externes Heizgerät: | Kälte-Umwälz- | Haake |
|  | Bad-Thermostat | Dieselstr. 4 |
|  | KT 2 | D-7500 Karlsruhe 41 |
|  | Typ 001-3973 |  |
| Internes Heizgerät: | Temperierkorb | BCC/IBT |
|  | eigene Entwick- | IBT e.V. |
|  | lung | Kellnerweg 6 |
|  |  | D-3400 Göttingen |
| Rührung: | Modul SPE | BCC |
| Interner Antrieb: | im Gefäßdeckel |  |
|  | fest verankert |  |
|  | Magnetrührstab |  |
|  | mit aufgesetzten |  |
|  | Rührpropellern |  |

5

(Fortsetzung)

Hersteller:

| | | |
|---|---|---|
| Externer Antrieb: | im Versorgungsgehäuse fest installierter Magnetrührer | |
| pH-Regulierung: | Modul PH | BCC |
| Elektrode: | pH-Elektrode autoklavierbar pH-Bereich 0-12 Typ 465-36-90-K9 | Ingold Siemensstr. 9 D-6374 Steinbach |
| pO$_2$-regulierung: | Modul PO$_2$ | BCC |
| Elektrode: | pO$_2$-Elektrode autoklavierbar Typ 7455 / 322756702 | Ingold |
| Gasversorgung: | | |
| Version I: | -Durchflußregelventile Meßrohr FD-I/8-08-G-5/m | Fischer & Porter Drensfelder Str.2 D-3400 Göttingen |
| | -Magnetventile Oliven 5 mm ø | BCC |
| | -Gasmischgefäß | BCC |
| Version II: | -Massendurchflußregler | MKS Schatzbogen 43 D-8000 München 82 |
| | -Gasmischgefäß | BCC |

6

(Fortsetzung)

Hersteller:

| | | |
|---|---|---|
| Druckminderer:<br>Preßluft: | Typ Nr. D 142/O$_3$<br>kontinuierlicher<br>Luftstrom | Drägerwerk AG<br>Moislinger<br>Allee 53/55<br>D-2400 Lübeck 1 |
| Hohlfaser<br>zur Belüftung: | Accurel (R) PP | Enka-Membrana<br>ENKA AKZO AG<br>Öhderstr. 28<br>D-5600 Wuppertal 2 |
| Porengröße: | Typ S 6/2 hydrophob<br>max. 0,54 μm | Akzo |
| Wicklung: | senkrecht zum Flüssig-<br>keitsspiegel | |
| Bedarf: | 3 m/l 1 Flüssigkeit | |
| Niveauregulierung: | Modul NIV | BCC |
| Elektroden: | autoklavierbar<br>Teflonüberzug | BCC |
| Dichtebestimmung<br>der Zell-Sus-<br>pension: | Durchflußphotometer | Monitec GmbH<br>Mörsenbroi-<br>cher Weg 200<br>D-4000 Düssel-<br>dorf 30 |

Beispiel 2

Kontinuierliche Produktion von ND (Newcastle Disease) Vakzine auf BHK 21 Zellen
Als Zellerhaltungsmedium wurde folgendes Medium verwendet:

| | |
|---|---|
| MEM (minimal ess. media) mit Earl's Spinnersalzen | 9,543 g/l |
| NaHCO$_3$ | 2,2 g/l |
| MEM-Vitaminlösung | 10,0 ml/l |
| fötales Kälberserum | 2,0 %/l |

7

Sterilisation des Mediums erfolgt durch Filtration über direkt an den Bioreaktor angeschlossene Hohlfaserbündel.

## Virusinoculum für den Bioreaktor B (Virus)

Der Virusfermenter wurde mit einer auf BHK Monolayer gezüchteten Kultur, die in 100 ml 7,1 x $10^7$ ID Virus enthält, beimpft. Die Infektion der Monolayerkultur war zwei Tage vor der ersten Frischzellenzugabe in den Bioreaktor erfolgt. Der Zellrasen der Monolayerkultur durfte bis zu diesem Zeitpunkt der Beimpfung des Virusfermenters noch keine Zellysis aufweisen. Die eigentliche Freisetzung der Viren sollte im Fermenter erfolgen und zwar zum Zeitpunkt der Zellzugabe aus dem Zellreaktor (Reaktor A).

Zur Inokulation aus Bioreaktor A wurden 6 x $10^6$ Zellen/ml in einer Durchflußmenge von 50 bis 100 ml pro Stunde (je nach Zellwachstum) von Bioreaktor A zu Reaktor B bei 3,0 x $10^8$ bis 6,0 x $10^8$ Zellen pro Stunde in Reaktor B zugegeben. Das Reaktorvolumen des Bioreaktor B war ebenfalls 4 l, das Flüssigkeitsvolumen 3 l, die Temperatur 37° C, die Rührgeschwindigkeit 150 Umdrehungen pro Minute, der pH-Wert wurde auf 7,0, der pO2-Wert auf 40,0 % $O_2$ in einer luftkalibrierten Lösung eingestellt. Der Gasgrundstrom betrug 4,0 l pro Minute, als Vordruck wurde 40 mbar $N_2$, 20 mbar $O_2$, 30 mbar $CO_2$ und Luft ohne Regelung des Vordrucks eingesetzt. Die Mediumzugabe betrug 2 l pro Tag und die Zellzugabe durchschnittlich 1,2 x $10^{10}$ Zellen pro Tag. Die Virusernte ergab 1,0 x $10^7$ ID/ml = 2,0 x $10^{10}$ ID/Tag, wobei die Ernte erfolgte, wenn 80 % der Zellen zerstört waren. Die Geräteausstattung des Bioreaktors 2 entsprach der des Bioreaktors I, jedoch mit folgenden Besonderheiten:

## Regulierung der Mediumzugabe in Verbindung mit dem Zellzulauf aus dem Reaktor I

Die im Reaktor I eingebaute Niveauregulierung öffnete zeitverzögert zwei Magnetventile:
Magnetventil 1: Zellfluß zu Reaktor II wurde ermöglicht
Magnetventil 2: Magnetventil 1 war geschlossen; zum Freispülen der Zuleitung von Zellen wurde der Mediumzulauf für Reak tor II geöffnet;
Öffnungszeit 5 bis 10 Sekunden

| Abluftentsorgung: | | |
|---|---|---|
| | | Firmen |
| Fritte: | 0,4 μm Porengröße | BCC |
| NaOH-Bad: | 2 %iges Laugenbad | |
| Filter: | Schwebstoffklasse S | Enka |
| | Porengröße 0,05 μm | Akzo |
| | hydrophob | |
| Bestimmung des cytopatischen Effektes zur Ermittlung des Virustiters: | Durchfluß-Photometer | BCC |

Die Abscheidung von Zellresten erfolgte über Zellfilter mit 0,45 nm Porengröße (Akzo MD 020 P2N) in der Filtrationsanordnung 1 und über Virusfilter 50.000 NMGG (Akzo V 100 I) in der Filtrationsanordnung 2. Zahnradpumpen, Niveauregulierung und Magnetventile stammten von der Firma BCC. Als übergeordnete Koordination der Bioreaktoren und Filter wurde der industrielle Leitrechner FLS1 mit angepaßter Software der Firma Münzer und Diehl verwendet. Das Leitrechnersystem kontrollierte durch Dichtebestimmung das Zellwachstum im Bioreaktor A und die Virusvermehrung über den cytopathischen (lytischen) Effekt in Bioreaktor B und steuert den Nährbodenzulauf zu Bioreaktor A und Zellabgabe von Bioreaktor B optimal. Die variabel drehenden Pumpen des Filtrationssystems wurden über den Rechner so gesteuert, daß alles anfallende Material sofort aufgearbeitet wurde.

**Ansprüche**

1. Kontinuierliches Verfahren zur Herstellung biologischer Materialien die nach Induktion von Zellkulturen herzustellen sind, dadurch gekennzeichnet, daß in einem ersten Bioreaktor eine Vorkultur erfolgt, das dann die Zellen in einem zweiten Bioreaktor kontinuierlich überführt werden und das bei oder nach der Überführung in den zweiten Bioreaktor die Induktion gesetzt wird, die zur Produktion des biologischen Materials führt.

2. Verfahren nach Anspruch 1 wobei die Induktion durch einen Virus, durch chemische oder durch physikalische Veränderungen erfolgt.

FIG.1

Zubereitung
Medien und
Waschlösung

Abluft

Gase

Gase

EP 0 417 531 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | AT - B - 374 828 (DR. KARL THOMAE GMBH) * Anspruch; Beispiel 1 * -- | 1,2 | C 12 N 5/00 C 12 N 5/02 //C 12 M 3/00 |
| A | AT - B - 370 132 (DR. KARL THOMAE GMBH) * Anspruch 1 * -- | 1,2 | |
| A | AT - B - 362 334 (THE WELLCOME FOUNDATION LTD.) * Anspruch 1; Beispiele 1,2 * -- | 1,2 | |
| A | EP - A1 - 0 301 833 (MITSUI PETROCHEMICAL INDUSTRIES, LTD.) * Zusammenfassung * ---- | 1 | |

| | | | RECHERCHIERTE SACHGEBIETE (Int Cl⁵) |
|---|---|---|---|
| | | | C 12 N C 12 M C 12 P |

Der vorliegende Recherchenbericht wurde für alle Patentansprüche erstellt.

| Recherchenort | Abschlußdatum der Recherche | Prüfer |
|---|---|---|
| WIEN | 06-11-1990 | WOLF |